# EUROPEAN PATENT SPECIFICATION

(11) **EP 1 155 692 B1**
(45) Date of publication and mention of the grant of the patent: **26.05.2004**
(21) Application number: 01110509.5
(22) Date of filing: 27.04.2001
(51) Int. Cl.: A61K 31/335, A61K 9/16, A61K 9/50, A61K 9/51, A61K 47/42

(54) **Use of Paclitaxel stabilized with albumin for preparing a drug for the treatment of solid tumors and the drug obtained thereby**
Verwendung von albuminstabilisiertem Paclitaxel zur Herstellung eines Arzneimittels zum Behandeln von festen Tumoren und das so erhaltene Arzneimittel
Utilisation de Paclitaxel stabilisé avec de l'albumine pour la préparation d'un médicament pour le traitement des tumeurs solides et médicament ainsi obtenu

(30) Priority: 18.05.2000 IT MI001107
(43) Date of publication of application: 21.11.2001
(73) Proprietor: ACS DOBFAR S.p.A., 20067 Tribiano (Milano) (IT)
(72) Inventor: Falciani, Marco, 20142 Milano (IT)
(74) Representative: Frignoli, Luigi

(56) References cited:
- WO-A-00/06152
- WO-A-94/18954
- WO-A-98/14174

## Description

The invention relates to a drug for the treatment of solid tumors sensitive to Paclitaxel, and the use of albumin microparticles incorporating Paclitaxel (and known by the name of ABI 007) in the preparation of this drug.

Paclitaxel is a substance well known in the literature, with important clinical activity on a large number of tumors, such as ovary, lung, head and neck, bladder and breast tumors.

Paclitaxel is insoluble in water. In order to render it soluble and suitable for intravenous administration, it has been mixed with a surfactant (such as polyethoxylated castor oil, known by the registered name "Cremophor EL") and with about 50% of anhydrous alcohol USP, as vehicles for the Paclitaxel: this mixture was patented by Bristol-Myers Squibb and is known by the registered name "Taxol" to which reference will be made hereinafter, for simplicity. The presence of the surfactant and the anhydrous alcohol has serious drawbacks, such as strong hypersensitivity, in addition to other side effects.

In any event, Taxol is administered, and can be administered, only by intravenous injection, with very lengthy administration times in an attempt to minimize the toxic effects of the product.

To overcome the aforesaid drawbacks, the said Bristol-Myers Squibb (BMS) has patented (European Patent Applications EP-A-0584001, EP-A-0783885, EP-A-0783886 and United States Patents US 5641803 and US 5670537) compositions also containing, in addition to Taxol, other substances or medicaments able to impede serious anaphylactic reactions.

According to the known art, as described in the aforesaid patents, from 135 to 175 mg of Taxol per m² of the patient's body surface are administered intravenously to patients subjected to therapy, each administration being effected very slowly, over a duration of about 3 hours: it is essential always to use the aforementioned medicaments.

US 5439686, US 5498421, US 5560933 and the corresponding WO 94 18954 describe albumin microparticles incorporating the Paclitaxel: these particles are internationally identified by the symbols ABI 007, this denomination being used hereinafter for simplicity whenever reference is made to these microparticles.

ABI 007 is in practice a formulation of Paclitaxel stabilized by human albumin USP, which can be dispersed in physiological solution and thus be directly injectable with very high concentrations of Paclitaxel being free of toxic emulsifiers.

The WO 94 18954 however gives no specific indications or examples as to which the concentration of ABI 007 in the solution should be in order to be safely and suitably administered as a drug to a patient for curing a tumor sensitive to Paclitaxel.

The later filed WO 98 14174 improves the teachings of the WO 94 18954 and gives an indication - only one example, Example 20 - as to which the concentration of ABI 007 in its solution should be for preparing a drug directly injectable to a patient for treating a tumor: such drug should consist of a dispersion of ABI 007 (named "CAPXOL" in the citation) in a physiological solution in which ABI 007 should be present at a very high concentration of 20 mg/ml of the solution, the infusion volume for a total dose of 200-500 mg being consequently so small (10-25 ml) that it is administered by a rapid intravenous bolus in a little as few minutes.

It has been surprisingly found that ABI 007 can be used to prepare drugs in which the ABI 007 is present as a dispersion in physiological solutions at a concentration between 2 mg and 8 mg per ml of these solutions, and that this drug can be injected directly into those arteries which feed the territory associated with a tumor sensitive to Paclitaxel, with administrations effected over short durations of many minutes and repeated just a few times.

Compared with the teachings of the aforestated previous known BMS patents, it can be noted that, according to the present invention, the ABI 007 is used to obtain drugs the use of which enables patients to be administered with doses of Paclitaxel very much higher and with infusion durations very much shorter than is possible with the known BMS teachings, with favourable results (response percentage extremely high and surprising), observing very low toxicity in all cases.

Compared with the teachings of the WO 98 14174, it can be noted that according to the present invention the ABI 007 is used for preparing a drug in which it is dispersed in a physiological solution at a concentration of 2 to 8 mg/ml of the solution and consequently with a toxicity which is much smaller than those enabled by the WO 98 14174, so that the drug can be injected over a duration of many minutes directly into the artery feeding the territory associated with the tumor and the injection can be repeated several times.

More specifically, the intra-arterial administrations of the drug of the invention can be spaced apart by 3-4 weeks, with a therapeutically very favourable result, i.e. a treatment response percentage which is higher than 71% (compared with the 40% achieved with the traditional treatment with Paclitaxel, for example in treating head and neck cancer).

More particularly, the drug of the present invention can be used with surprising results for treating a patient afflicted with a tumor sensitive to Paclitaxel, this drug being injectable directly into the artery feeding the territory associated with the tumor, taking care to use a quantity of drug containing between 190 and 500 mg of ABI 007 per m² of the patient's body and that the injection is completed over a duration of about 25-60 minutes. From 2 to 5 similar intra-arterial injections are then repeated at a distance of 3-5 weeks one from another.

Tumors which have proved responsive to the described treatment are the squamocellular carcinomas, including certain tumors of the lung, head and neck, uterus and anal canal.

From trials carried out it has been found that, preferably, the drug quantity used for intra-arterial therapy of tumors contains from 200 to 300 mg of ABI 007 per m² of the patient's body surface for each administration, and that the administration of each individual dose of the dispersion is effected over 30 minutes.

Some examples relating to the treatment of tumors of different types and locations are described to clarify the understanding and the characteristics of the present invention.

### EXAMPLE 1

A female patient (PR), 57 years of age, afflicted with pelvic recurrence of carcinoma of the anal canal, already previously treated by chemotherapy, radiotherapy and surgery and judged inoperable, is treated with four separate successive intra-arterial injections (at a distance of 4 weeks one from the other) into the internal iliac arteries, of dispersions of ABI 007 in physiological solution (Paclitaxel in microparticles of albumin) at doses of 220, 230, 250 and 275 mg per m² of body surface respectively.

The injected dispersions have a concentration of 6 mg of ABI 007 per ml of their solution.

The reduction in the tumor was followed by computerized tomography. Having observed an important tumor reduction and assuming that virtually complete regression of the mass had been achieved, a surgical intervention was performed and demonstrated the absence of the histological tumor.

### EXAMPLE 2

To a female patient (ZN), 64 years of age, afflicted with voluminous neoplasia of the left half tongue, not previously subjected to any form of treatment, ABI 007 is administered in 4 successive cycles, at a dose of 210, 230, 200 and 200 mg/m² respectively, at a concentration of 4 mg/ml into the left lingual artery.

Response to the treatment was followed by clinical examination and magnetic resonance because of the presence of fixed dental prostheses.

Having observed important tumor regression, evaluated at 90% one month after the end of treatment, surgical intervention was proposed, and demonstrated histological absence of the tumor.

### EXAMPLE 3

To a male patient (BP), 61 years of age, afflicted with carcinoma of the right hypopharynx, with metastasis at a right laterocervical lymph node not subjected to any previous treatment, ABI 007 is administered into the external carotid artery for 3 cycles, spaced apart by 4 weeks, at a dose of 240 mg/m² and at a concentration of 3 mg/ml. Having observed the therapeutic response by computerized tomography and clinical examination, multiple depth biopsies were performed in the hypopharynx, in the site previously occupied by the tumor, and demonstrated histological absence of neoplasia.

The lymphonodal metastasis had also decreased by more than 80% in dimensions and was removed by surgical intervention, with tumor presence at this level.

### EXAMPLE 4

To a female patient (EP), 37 years of age, afflicted with recurrence of carcinoma of the anal canal, previously treated by radio- chemotherapy, ABI 007 was administered into the internal iliac arteries for 4 cycles spaced apart by 4 weeks, at a dose of 250 mg/m² per cycle, at a concentration of 7 mg/ml.

Having observed the therapeutic response by gynecological examination and computerized tomography, a new surgical intervention was proposed and demonstrated the histological absence of tumor.

### EXAMPLE 5

To a male patient (GC), 59 years of age, afflicted with recurrence of carcinoma of the anal canal, previously treated by radio-chemotherapy, ABI 007 was administered into the internal iliac arteries at a dose of 200, 240 and 260 mg/m² respectively, at a concentration of 7.5 mg/ml.

Having observed the response by clinical examination and transrectal echoendosonography, a surgical intervention was proposed and demonstrated the histological absence of tumor.

## Claims

1. Use of microparticles of Paclitaxel stabilized with albumin for preparing a drug for the treatment of solid tumors sensitive to Paclitaxel, in particular squamocellular carcinomas, **characterized in that** said microparticles are dispersed in a physiological solution in which the microparticles are present at a concentration between 2 and 8 mg/ml of such solution.

2. Use of microparticles as claimed in claim 1, **characterised in that** the concentration of said microparticles in their dispersion is between 3 and 7.5 mg/ml of the physiological solution.

3. A drug for the treatment of solid tumors sensitive to Paclitaxel, comprising a dispersion of microparticles of Paclitaxel stabilized with albumin in a physiological solution **characterised in that** the concentration of the microparticles is between 2 and 8 mg/ml of said solution.

4. A drug as claimed in claim 3, **characterised in that** in said dispersion the concentration of the microparticles is between 3 and 7.5 mg/ml of the solution.

## Patentansprüche

1. Verwendung von Paclitaxel-Mikroteilchen, die mit Albumin stabilisiert sind, zur Herstellung eines Arzneimittels zur Behandlung fester, Paclitaxel-empfindlicher Tumore, insbesondere von Plattenepithelkarzinomen, **dadurch gekennzeichnet, dass** die Mikroteilchen in einer physiologischen Lösung dispergiert werden, in der die Mikroteilchen in einer Konzentration zwischen 2 und 8 mg/ml derartiger Lösung vorliegen.

2. Verwendung von Mikroteilchen nach Anspruch 1, **dadurch gekennzeichnet, dass** die Konzentration der Mikroteilchen in ihrer Dispersion zwischen 3 und 7,5 mg/ml der physiologischen Lösung ist.

3. Arzneimittel zur Behandlung fester, Paclitaxel-empfindlicher Tumore, umfassend eine Dispersion von Paclitaxel-Mikroteilchen, die mit Albumin stabilisiert sind, in einer physiologischen Lösung, **dadurch gekennzeichnet, dass** die Konzentration der Mikroteilchen zwischen 2 und 8 mg/ml der Lösung ist.

4. Arzneimittel nach Anspruch 3, **dadurch gekennzeichnet, dass** in der Dispersion die Konzentration der Mikroteilchen zwischen 3 und 7,5 mg/ml der Lösung ist.

## Revendications

1. Utilisation de microparticules de Paclitaxel stabilisées à l'albumine pour préparer un médicament pour le traitement de tumeurs solides sensibles au Paclitaxel, en particulier des carcinomes squamocellulaires, **caractérisée en ce que** lesdites microparticules sont dispersées dans une solution physiologique dans laquelle les microparticules sont présentes à une concentration de 2 à 8 mg/ml de cette solution.

2. Utilisation de microparticules selon la revendication 1, **caractérisée en ce que** la concentration desdites microparticules en dispersion est de 3 à 7,5 mg/ml de la solution physiologique.

3. Médicament pour le traitement de tumeurs solides sensibles au Paclitaxel, composé d'une dispersion de microparticules de Paclitaxel stabilisées à l'albumine dans une solution physiologique, **caractérisé en ce que** la concentration des microparticules est de 2 à 8 mg/ml de ladite solution.

4. Médicament selon la revendication 3, **caractérisé en ce que**, dans ladite dispersion, la concentration des microparticules est de 3 à 7,5 mg/ml de la solution.
